Europäisches Patentamt

European Patent Office

Office européen des brevets

(19)

(11) Numéro de publication : **0 391 803 B1**

(12) **FASCICULE DE BREVET EUROPEEN**

(45) Date de publication du fascicule du brevet :
**29.07.92 Bulletin 92/31**

(21) Numéro de dépôt : **90400931.3**

(22) Date de dépôt : **04.04.90**

(51) Int. Cl.$^5$ : **B01J 13/08,** A61K 7/00,
A61K 9/50

(54) **Procédé de préparation de capsules d'alginate(s), particulièrement adaptées à un usage cosmétique, appareil pour sa mise en oeuvre et composition cosmétique contenant lesdites capsules.**

(30) Priorité : **07.04.89 FR 8904598**

(43) Date de publication de la demande :
**10.10.90 Bulletin 90/41**

(45) Mention de la délivrance du brevet :
**29.07.92 Bulletin 92/31**

(84) Etats contractants désignés :
**AT BE CH DE DK ES FR GB GR IT LI NL SE**

(56) Documents cités :
**GB-A- 1 236 885**
**A. KONDO: "Microcapsule Processing and Technology", 1979, pages 65-66, Marcel Dekker, Inc., New York, US**

(73) Titulaire : **L'OREAL**
**14, Rue Royale**
**F-75008 Paris (FR)**

(72) Inventeur : **Handjani, Rose-Marie**
**17Bis, rue Campagne Première**
**F-75014 Paris (FR)**
Inventeur : **Kauffmann, Myriam**
**25, boulevard des Belges**
**F-69006 Lyon (FR)**
Inventeur : **Huguenin, Frédéric**
**8, rue du Four**
**F-75006 Paris (FR)**

(74) Mandataire : **Peuscet, Jacques et al**
**Cabinet Peuscet 68, rue d'Hauteville**
**F-75010 Paris (FR)**

**EP 0 391 803 B1**

## Description

La présente invention concerne un procédé de préparation de capsules d'alginate(s) particulièrement adaptées à un usage cosmétique. Elle concerne également l'appareil permettant la mise en oeuvre du procédé ainsi qu'une composition cosmétique contenant les capsules préparées par ledit procédé.

Les alginates sont des produits connus ; ils sont constitués par des chaînes de polysaccharides obtenues par enchaînement de deux types de monomères, l'acide guluronique (G) et l'acide mannuronique (M) qui s'organisent en blocs G, blocs M, et blocs à monomères alternés G-M. La gélification s'effectue par pontage de ces chaînes polysaccharidiques à l'aide d'ions polyvalents. Ce pontage s'effectue préférentiellement entre les blocs G.

La fabrication de capsules d'alginate(s) par gélification des solutions aqueuses de sels solubles d'alginate(s), tels que les sels de potassium, de magnésium, d'ammonium et préférentiellement de sodium, ou des solutions aqueuses de leurs dérivés, tels que leurs esters partiels, au moyen de certains cations métalliques polyvalents, en particulier le calcium, est connue. Pour préparer des capsules d'alginate(s), on fait tomber la solution d'alginate(s), goutte à goutte à l'aide d'une buse, dans une solution de sel de métal polyvalent. Les capsules obtenues sont ensuite séparées du milieu réticulant.

Pour que les capsules obtenues puissent être utilisées en cosmétique, il est nécessaire qu'elles aient à la fois les deux propriétés antagonistes suivantes : s'écraser facilement sur la peau, sans laisser de résidu, sous l'action d'un léger massage, et avoir une rigidité suffisante pour que leur structure ne soit pas modifiée au cours de leur stockage ou de leur incorporation dans une composition cosmétique.

La Demanderesse a trouvé, selon la présente invention, qu'en ajustant de façon précise les conditions de fabrication des capsules d'alginate(s), en particulier les conditions cinétiques de réticulation au cours de la fabrication, on pouvait obtenir des capsules d'alginate(s) ayant les propriétés requises pour un usage cosmétique.

La présente invention concerne un procédé de fabrication de capsules d'alginate(s) par introduction goutte à goutte d'une solution aqueuse d'au moins un alginate dans une solution aqueuse d'au moins un sel de métal polyvalent choisi parmi le fer, l'argent, le strontium, l'aluminium, le manganèse, le sélénium, et en particulier le calcium, le cuivre et le zinc, à l'aide d'au moins une buse, puis extraction des capsules d'alginate(s), formées par gélification, pour les séparer de la solution aqueuse de sel(s) de métal polyvalent et enfin, éventuellement lavage et séchage desdites capsules, caractérisé par le fait que :

a) on utilise au moins un alginate ayant des unités mannuroniques (M) et guluroniques (G) dans un rapport molaire M/G compris entre $\emptyset,4$ et 1,9 et, de préférence, une proportion de blocs (G) supérieure à $5\emptyset$ %, le (ou les) alginate(s) étant, de préférence un (ou des) alginate(s) de sodium ayant une viscosité en solution aqueuse à $\emptyset,5$ % en poids à 25°C inférieure à $2\emptyset$ mPa.s mesurée au viscosimètre TV Contraves avec un corps de mesure n° 1 en présence d'un chélatant du calcium ;

b) la concentration pondérale en alginate(s) de la solution aqueuse introduite par la (ou les) buse(s) est comprise entre $\emptyset,2$ et 2 % de préférence entre $\emptyset,3$ et 1 % ;

c) la concentration molaire en cation métallique polyvalent de la solution aqueuse réticulante de sel(s) de métal polyvalent est de $3,4 \times 1\emptyset^{-3}M$ à $6,8 \times 1\emptyset^{-2}M$, de préférence $6,8 \times 1\emptyset^{-3}M$ à $3,4 \times 1\emptyset^{-2}M$;

d) la tension superficielle de la solution aqueuse de sel(s) de métal polyvalent est abaissée à une valeur inférieure à $7\emptyset$ dynes/cm, de préférence voisine de $4\emptyset$ dynes/cm, par addition d'un agent tensio-actif ; et

e) le temps de contact entre les gouttes de solution d'alginate(s) et la solution aqueuse de sel(s) de métal polyvalent est un temps précis compris entre $1\emptyset$ secondes et $2\emptyset$ minutes, de préférence entre $3\emptyset$ secondes et $1\emptyset$ minutes.

Dans ces conditions, on obtient des capsules d'alginate(s) sphériques, sphéroïdales, ovales ou en forme de gouttes ou de grains de riz, ayant une texture adaptée à l'usage cosmétique ; quand elles sont sphéroïdales, elles peuvent avoir un diamètre compris entre $1\emptyset\emptyset$ μm et 1 cm et de préférence $5\emptyset\emptyset$ μm et $\emptyset,8$ cm.

Selon la présente invention la solution aqueuse d'alginate(s) peut contenir au moins un additif ou actif cosmétique hydrosoluble ou dispersible. Ladite solution peut éventuellement contenir des additifs ou actifs liposolubles, ou, des solides organiques ou minéraux qui resteront en dispersion ou en suspension dans la matrice alginique. Parmi les actifs utilisables, on peut citer des composés biologiques, des extraits végétaux ou animaux, des pigments colorés ou non, des charges minérales, des filtres solaires, des polymères solubles ou insolubles, des eaux florales, des huiles essentielles, des compositions parfumées, des substances pour l'hygiène buccale et les soins dentaires, des cristaux liquides de type cholestérique, des dispersions vésiculaires, des microsphères ou des microcapsules polymériques ou lipidiques, des nanoparticules ou des nanocapsules. Les actifs utilisés couramment pour l'hygiène buccale ou pour les soins dentaires peuvent être introduits dans la solution aqueuse d'alginate(s) ou dans la phase externe de la composition contenant les capsules d'alginate. Ces actifs sont par exemple :

2

– des agents antiseptiques, antiinflammatoires, décongestionnants ou adoucissants (salicylate de choline, extraits végétaux, acide glycérrhétinique...),
– des agents de captation des mauvaises odeurs ou des molécules issues des débris alimentaires (cyclodextrines, ricinoléate de zinc...),
– des agents de polissage (poudres minérales),
– des substances pouvant agir sur la partie minérale de la dent (sels de strontium...).

La solution aqueuse d'alginate(s) peut également contenir une faible quantité d'ions réticulants destinés à modifier ses propriétés rhéologiques.

La solution de sel(s) de métal polyvalent utilisée est, de préférence, une solution de chlorure, de gluconate ou de lactate de calcium ou une solution d'acétate, de sulfate, de chlorure ou de gluconate de cuivre ou de zinc. Le volume de la solution réticulante doit, en général, être suffisant pour que celle-ci ne soit pas épuisée en métal polyvalent ou ne soit appauvrie que dans des proportions négligeables au cours de la formation des capsules. De plus la solution ne doit pas être animée de turbulences, qui provoqueraient la distorsion des capsules en cours de formation.

De plus, la densité de la solution d'alginate(s) doit, de préférence, être au moins légèrement supérieure à celle de la solution des cations polyvalents, de façon à ce que les gouttes en tombant, soient entraînées vers le bas.

Selon l'invention, on ajoute à la solution de sel(s) de métal polyvalent un agent tensio-actif ; cet agent tensio-actif peut être, par exemple, le produit commercialisé sous la dénomination "TWEEN 2Ø" par la Société I.C.I. AMERICAS à une concentration pondérale de Ø,ØØ1 %.

Selon la présente invention, on peut utiliser une ou plusieurs buse(s) pour l'introduction de la solution aqueuse d'alginate(s) dans la solution de sel(s) de métal polyvalent. Le diamètre des capsules obtenues dépend de la dimension des buses utilisées. Par exemple, avec une buse de Ø,6 millimètre de diamètre, on forme des sphères de 1,5 à 2,5 millimètres et avec une buse de 1,5 mm de diamètre des sphères de 3 à 6 millimètres.

La solution d'alginate(s) peut être délivrée au-dessus de la solution de sel(s) de métal polyvalent. La hauteur de la (ou des) buse(s) au-dessus de la surface de la solution réticulante doit être alors, de préférence, juste suffisante pour que les gouttes se forment et se séparent de la buse sous l'effet de leur propre poids, ou par cisaillement forcé. Si elle est trop importante, les gouttes sont déformées à leur arrivée dans la solution réticulante et perdent leur forme sphérique.

La solution d'alginate(s) peut également être injectée sous la surface de la solution réticulante ; dans ce cas, si un mouvement est imprimé à la buse, on peut obtenir des particules de formes différentes. Par exemple un mouvement linéaire formera des capsules allongées.

Le procédé selon la présente invention se déroule de la façon suivante : on fait tomber la solution d'alginate(s) contenant aussi le(s) éventuel(s) actif(s) en solution ou en dispersion, goutte à goutte à l'aide d'une ou plusieurs buses dans la solution réticulante. Les gouttes sont ainsi gélifiées et forment des capsules. Après un temps de séjour chronométré de façon précise, les capsules sont extraites du milieu de réticulation, par tamisage ou égouttage par exemple. On élimine ensuite, éventuellement par rinçage à l'eau, de préférence déminéralisée, les ions réticulants excédentaires. L'eau libre à la surface des capsules est ensuite éventuellement éliminée par séchage ou égouttage. Les capsules, après rinçage et séchage, peuvent être enrobées par une couche d'un polymère en solution ou en suspension, plus ou moins adhésif. Cet enrobage est obtenu, par exemple, en trempant les capsules dans une solution ou une suspension d'un polymère acrylique adhésif, telle que celles vendues par la Société MONSANTO sous les dénominations commerciales : "GELVA multipolymer emulsion 2397" et "GELVA multipolymer emulsion 3Ø11".

La présente invention a également pour objet une composition cosmétique contenant les capsules d'alginate(s) préparées par le procédé décrit précédemment dans une phase externe cosmétiquement acceptable. Dans cette composition cosmétique, la phase externe peut être un gel, une émulsion, une solution, une huile ou un excipient huileux plus ou moins transparent ou translucide, cosmétiquement acceptable. La phase externe peut aussi renfermer tout type d'additif ou d'actif au sens cosmétique.

La présente invention a également pour objet un appareil pour la mise en oeuvre du procédé selon l'invention comportant un réservoir de stockage de la solution d'alginate(s), au moins une buse pour délivrer ladite solution d'alginate(s), une pompe prélevant la solution d'alginate(s) du réservoir de stockage et alimentant la (ou les) buse(s), un bac allongé alimenté en continu à une de ses extrémités, par une solution aqueuse de sel(s) du cation réticulant et comportant, à son autre extrémité, un dispositif de soutirage par exemple par trop-plein de ladite solution réticulante et éventuellement un système de régénération et recyclage de ladite solution, la solution d'alginate(s) tombant goutte à goutte de la (ou des) buse(s) dans la solution réticulante à l'extrémité du bac correspondant à l'alimentation en sel(s) de métal polyvalent, et un dispositif de transport disposé longitudinalement dans le bac et permettant de transporter les capsules d'alginate(s) en cours de formation d'une extrémité à l'autre du bac. Selon la présente invention le dispositif de transport peut être une vis sans fin ; cependant, il est, de préférence, constitué par une bande sans fin entraînée par un moteur. La bande sans fin est, de préférence, formée d'une trame ou d'un matériau flexible perforé, dont la maille est inférieure au diamètre des gouttes tombant de la buse. La bande peut être en polyamide ou autre polymère, en acier inoxydable ou en autre métal ; elle comporte, de préférence, des saillies transversales, qui empêchent le retour en arrière des capsules en cours de formation.

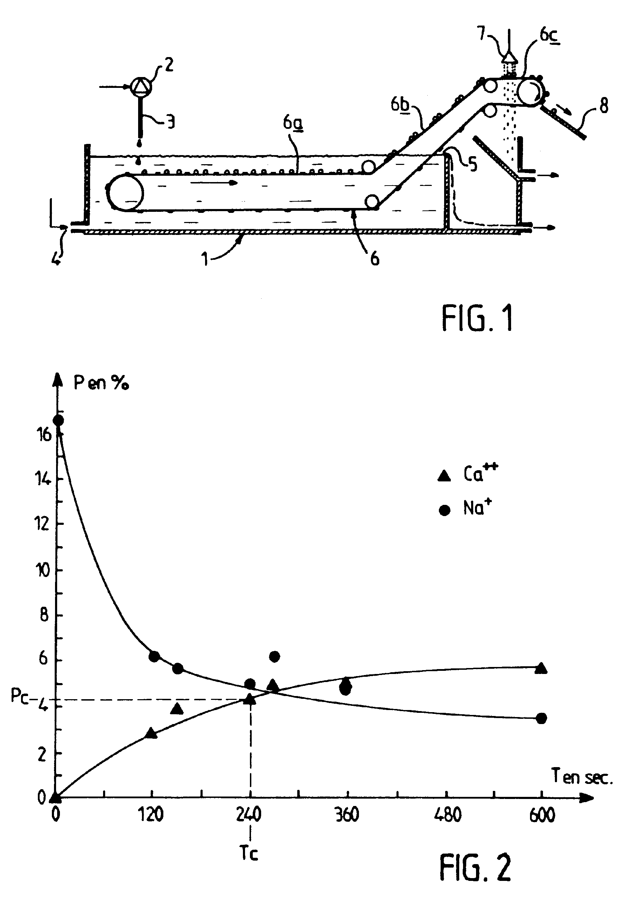

Sur le dessin, la figure 1 représente schématiquement, à titre purement illustratif et non limitatif, un appareil de ce type, industriellement utilisable.

En se référant au dessin, on voit que la solution aqueuse réticulante est contenue dans un bac 1 ; la solution d'alginate(s) est alimentée par l'intermédiaire d'une pompe 2 dans les buses 3 et tombe goutte à goutte dans la solution contenue dans le bac 1. Le bac 1 est alimenté en solution réticulante par une conduite 4, à une de ses extrémités et au voisinage de son fond ; ladite solution est évacuée par un trop plein 5 à l'autre extrémité.

Le bac 1 contient une bande sans fin 6, qui est constituée par une trame, par exemple en polyamide du type connu sous la marque "Nylon", ayant des mailles inférieures ou égales à un millimètre ; cette bande 6 comporte des saillies transversales ; elle est disposée longitudinalement dans le bac 1 et actionnée par un moteur (non représenté). La partie supérieure de la bande 6 passe au voisinage des buses 3. Sa partie inférieure sert au retour. La zone supérieure comporte une partie horizontale immergée 6a, suivie d'une partie inclinée ascendante 6b, elle-même prolongée par une partie horizontale 6c ; la partie inclinée 6b permet à la bande de sortir du bac 1. La vitesse de la bande sans fin 6 est ajustable pour que le temps de contact entre les capsules d'alginate(s) et la solution réticulante ait une valeur précise comprise entre 10 secondes et 20 minutes. A la sortie du bac 1, la bande sans fin entraîne les capsules d'alginate(s) formées, hors du bain. L'emploi d'une bande sans fin constituée par une trame maillée permet d'égoutter les capsules ; les saillies transversales empêchent les capsules de retomber en arrière en roulant dans la partie inclinée 6b. La présence des saillies transversales permet donc d'augmenter la pente du convoyeur à la sortie de la solution aqueuse de sel(s) de métal polyvalent et, par conséquent, de diminuer le dimensionnement de l'installation totale et de réduire le temps d'égouttage pendant lequel la réaction est mal contrôlée. Sur la partie horizontale 6c de la bande sans fin, les capsules d'alginate(s) sont aspergées d'eau déminéralisée grâce à une tête d'arrosage 7, pour éliminer les traces de sel(s) de métal polyvalent excédentaire(s).

Eventuellement, les capsules peuvent être traitées ensuite par un jet d'air (non représenté) qui les sèche en surface. Les capsules prêtes pour le conditionnement sont recueillies à la sortie 8 de la bande sans fin.

Les exemples donnés ci-dessous, à titre purement illustratif et non limitatif, permettront de mieux comprendre l'invention.

## EXEMPLE 1

On fait tomber 3 g d'une solution aqueuse d'alginates de sodium goutte à goutte dans une solution aqueuse réticulante obtenue par dissolution de chlorure de calcium $CaCl_2$, $2H_2O$. La solution d'alginates est délivrée par une buse de 1,3 millimètre de diamètre dont l'extrémité est 15 mm au-dessus du niveau de solution réticulante. On obtient des sphères d'environ 4 mm. On laisse les capsules formées en contact avec la solution réticulante pendant un temps déterminé ; on extrait par tamisage, on rince, on pèse et on dispose les capsules sur un filtre sans cendre taré. Le filtre et les sphères sont déshydratés et pesés, ce qui permet de déterminer le poids de résidu sec. On dose ensuite sur ce résidu sec les ions $Ca^{++}$ par spectrophotométrie d'absorption et les ions $Na^+$ par spectrophotométrie d'émission. On trace les courbes donnant, en fonction du temps de contact, le pourcentage de $Ca^{++}$ fixé et le pourcentage de $Na^+$ contenu dans les capsules d'alginate(s) ; en effet, la courbe de variation du pourcentage de $Na^+$ est une vérification du bon déroulement de l'échange sodium/calcium dans les alginates.

Par ailleurs, on apprécie sensoriellement pour les temps de contact variables, la résistance à l'écrasement des capsules obtenues. On détermine ainsi un temps de contact $t_c$ et un pourcentage en ions calcium $P_c$ pour lesquels on obtient la consistance cosmétique optimum des capsules.

Les essais ont été faits avec les alginates vendus par la Société "KELCO-FRANCE" sous la dénomination commerciale "Manugel GHB" ayant une viscosité d'environ 8 mPa.s en solution à 0,5 % (mesurée à 25°C au viscosimètre TV Contraves, avec un corps de mesure n° 1, en présence d'un chélatant du calcium et avec les alginates vendus par la Société "SIGMA" sous la dénomination commerciale "ALGINIC ACID SODIUM SALT TYPE VI moyenne viscosité (MV)" ayant une viscosité d'environ 17 mPa.s en solution à 0,5 % à 25°C mesurée au viscosimètre TV Contraves avec un corps de mesure n° 1 en présence d'un chélatant du calcium. On a ainsi obtenu, pour ces alginates, les courbes données sur les figures 2 à 5, dans lesquelles le temps de contact T en secondes est donné en abscisse et les pourcentages en poids P des ions $Ca^{++}$ et $Na^+$ par rapport au poids d'alginate sec sont portés en ordonnée.

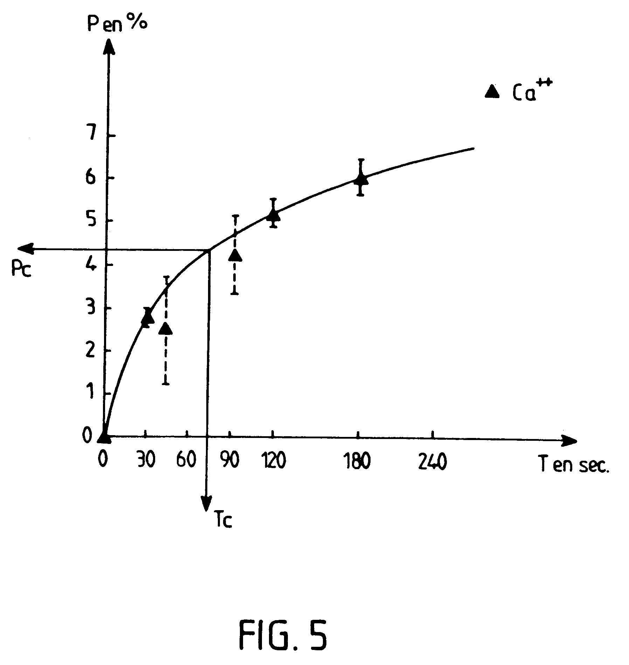

Les figures 2 à 4 concernent des sphères de 4 à 4,5 mm de diamètre (buse de 1,3 mm). La figure 5 concerne des sphéres de 2 à 2,5 mm de diamètre (buse de Ø,9 mm).

Sur ces courbes, on voit que, lorsque le temps de contact entre la solution d'alginates et la solution de chlorure de calcium croît, il se produit une fixation de Ca$^{++}$ rapide au départ puis lente et tendant vers la valeur théorique maximum de fixation du calcium. A l'inverse la concentration en Na$^+$ s'abaisse rapidement puis plus lentement.

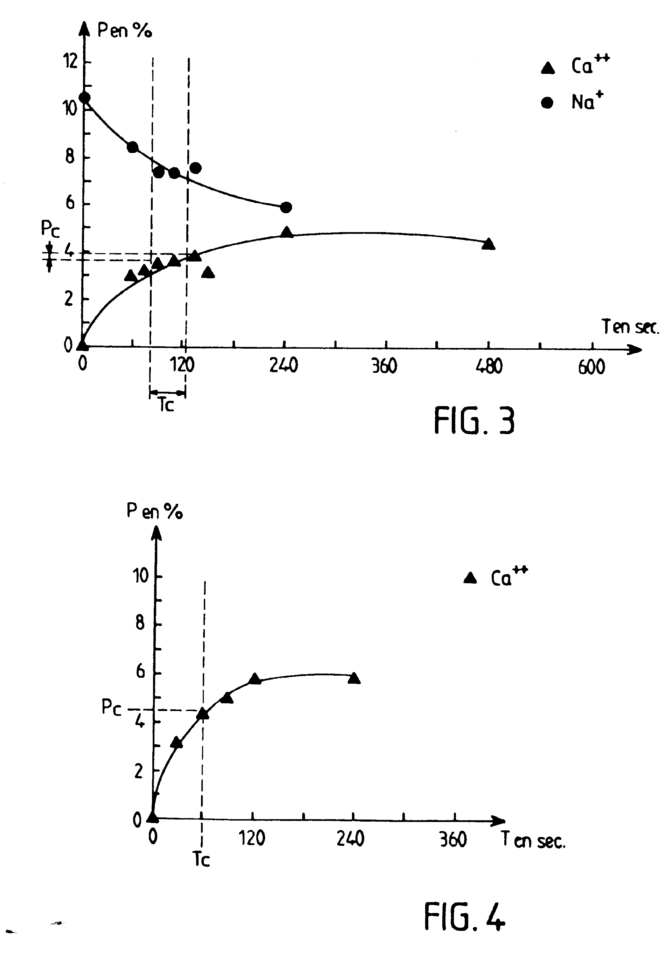

Les courbes des figures 2, 4 et 5 ont été tracées avec une solution aqueuse de "Manugel GHB" ayant une concentration de Ø,5 %, la courbe de la figure 4 ayant été tracée pour une solution réticulante ayant une concentration pondérale de Ø,5 % exprimée en (CaCl$_2$, 2H$_2$O) et les courbes des figures 2 et 5 pour une solution réticulante ayant une concentration exprimée en (CaCl$_2$, 2H$_2$O) de Ø,2 % en poids par rapport au poids de solution.

Dans ces trois essais, on a constaté que, lorsque la teneur pondérale en ions CA$^{++}$ dans les capsules est inférieure à 2,5 %, ces dernières sont fragiles, molles, et leur paroi est fine ; elles s'affaissent, se déforment et éclatent trop facilement. Au-dessus d'une teneur pondérale en ions Ca$^{++}$ de 5,5 %, les sphères sont dures, caoutchouteuses, pleines, difficiles à écraser et laissent des débris élastiques sur la peau. La consistance correspondant à un usage cosmétique se situe entre les deux. Dans ces essais, la quantité optimale en Ca$^{++}$ introduite par pontage dans les alginates est voisine de 4,5 % en poids par rapport au poids d'alginates déshydratés, cette concentration étant atteinte en 1 minute dans le cas de la figure 4, en 4 minutes dans le cas de la figure 2, et en 1 minute 15 secondes dans le cas de la figure 5.

La courbe de la figure 3 correspond à une solution aqueuse d'"ALGINIC ACID SODIUM SALT TYPE VI de moyenne viscosité" de la société "SIGMA" à Ø,35 % en poids et à une solution réticulante de (CaCl$_2$, 2H$_2$O) à Ø,2 % en poids. La quantité optimale de Ca$^{++}$ fixée par pontage, correspondant à des capsules d'alginates ayant une consistance cosmétique est de 3,6 à 3,9 % en poids par rapport au poids d'alginate déshydraté et correspond à un temps de contact de 1 minute 30 secondes à 2 minutes 15 secondes.

EXEMPLE 2

Des capsules d'alginates sont préparées dans un appareil du type illustré sur la figure 1 comportant des buses d'alimentation de 1,3 millimètre de diamètre émettant des gouttes à une fréquence de 1 Hertz. Le temps de contact entre la solution d'alginates A et la solution B de chlorure de calcium est de 2 minutes.

La solution aqueuse d'alginates A a la composition suivante :

```
dénomination commerciale "Sigma type VI" ..    Ø,35Ø  g

Glycérine ...............................    5,ØØØ  g

Extrait de Gingko Biloba dans du

propylène-glycol .......................    2,ØØØ  g

Conservateur ...........................    Ø,1ØØ  g

Eau déminéralisée ................. q.s.p.  1ØØ,ØØ   g
```

La solution réticulante B a la composition suivante :

```
CaCl₂, 2H₂0 ............................    Ø,2    g
Tensio-actif vendu sous la dénomination

commerciale "Tween 2Ø" par la Société

"ICI Americas" .........................    Ø,ØØ1 g

Eau déminéralisée ................. q.s.p.  1ØØ,ØØ   g
```

Les capsules sortant de la solution B sont lavées et séchées en surface. Les capsules obtenues sont des sphères d'un diamètre de 4 mm. Elles sont constituées par un gel transparent très doux, qui, après étalement sur la peau accompagné par une léger massage, sont absorbées totalement et très rapidement en laissant une sensation de fraîcheur et d'hydratation.

Ces sphères sont ensuite incorporées dans une formulation finale C ayant pour composition :

```
Capsules ................................    90      g
DL Alpha-tocophérol .....................     1      g
Parfum ............................ q.s.
Diméthyl polysiloxane cyclique
dénommé Cyclométhicone
dans le CTFA Cosmetic Dictionary,

3ème édition, publié par the
"Cosmetic Toiletry and Fragance
Association" .........................q.s.p.  100,00   g
```

On constate après 24 heures que les capsules ont conservé leur forme et individualité dans la formule.

EXEMPLE 3

On prépare des capsules d'alginates comme dans l'exemple 1 avec une buse d'injection de 1,3 millimètre de diamètre et un temps de contact entre les solutions A et B de 1 minute 15 secondes. La solution d'alginates A a pour formule :

Alginates de sodium vendus sous la dénomination commerciale

```
"Kelco Manugel GHB" .....................    0,50   g
Glycérine ...............................    4,00   g
Extrait placentaire aqueux de
Filatov .................................   20,00   g
Guanosine en cristaux ...................    0,01   g
Micatitane ..............................    0,10   g
Conservateur ...................... q.s.
Eau déminéralisée ................. q.s.p.  100,00   g
```

La solution réticulante a pour formule :

```
CuSO₄, 5H₂O .............................    0,85   g
Tensio-actif vendu sous la
dénomination "Tween 20" par
la Société "ICI Americas" ...............    0,001  g
Eau déminéralisée ................. q.s.p.  100,00   g
```

Les capsules obtenues sont des sphères d'un diamètre de 4,5 mm. On les incorpore dans une formule finale C ayant pour composition :

EP 0 391 803 B1

```
Capsules  ...................................    95      g
Huile d'amandes douces  ...................     2      g
Parfum  ............................. q.s.
Diméthyl polysiloxane cyclique
dénommé Cyclométhicone


dans le CTFA Cosmetic Dictionary,
3ème édition, publié par the
"Cosmetic Toiletry and Fragance
Association"  ...................... q.s.p.  100,00  g
```

On constate qu'après 24 heures, les capsules ont conservé leur forme et leur individualité dans la formule et qu'elles s'écrasent facilement sous le doigt.

<u>EXEMPLE 4</u>

On prépare des capsules d'alginates comme dans l'exemple 1 avec une buse d'injection de Ø,8 mm et un temps de contact entre les solutions d'alginates A et de sel de calcium B de 4 minutes.

La solution A a pour composition :

Alginates de sodium vendus sous la dénomination commerciale

```
"Sigma low vicosity"  ...................    Ø,50   g
Extrait aqueux d'algues brunes  ...........   10     g
Extrait aqueux d'algues rouges  ...........   10     g
Glycérine  ...............................   10     g
Hydroxyproline  ..........................    1     g
D-Panthénol (vitamine B5)  ...............    1,5   g
Conservateur  ...................... q.s.
Eau déminéralisée  ................. q.s.p.  100,00  g
```

La solution B a pour composition :

```
Zn(CH3COO)2,2H2O  .......................    Ø,3    g
Tensio-actif vendu sous la
dénomination commerciale "Tween 20"
par la Société ICI Americas  .............   Ø,001 g
Eau déminéralisée  ................. q.s.p.  100,00  g
```

Les capsules obtenues sont des sphères d'un diamètre de 2 mm. On les incorpore dans une phase externe. La formule finale C a la composition suivante :

```
Capsules ................................... 6Ø      g
Palmitostéarate de polyglycol ............. 5       g


Huile minérale ............................   3,2    g
Conservateur ...................... q.s.
Parfum ............................ q.s.
Eau déminéralisée ................. q.s.p.  1ØØ,ØØ  g
```

On constate qu'après 24 heures, les capsules ont conservé leur forme et leur individualité et qu'elles s'écrasent facilement sous le doigt.

**Revendications**

1. Procédé de fabrication de capsules d'alginate(s) par introduction goutte à goutte d'une solution aqueuse d'au moins un alginate dans une solution aqueuse d'au moins un sel de métal polyvalent choisi parmi le fer, l'argent, le strontium, l'aluminium, le manganèse, le sélénium, et en particulier le calcium, le cuivre et le zinc, à l'aide d'au moins une buse, puis extraction des capsules d'alginate(s), formées par gélification, pour les séparer de la solution aqueuse de sel(s) de métal polyvalent et enfin, éventuellement lavage et séchage desdites capsules, caractérisé par le fait que :
a) on utilise au moins un alginate ayant des unités mannuroniques (M) et guluroniques (G) dans un rapport molaire M/G compris entre Ø,4 et 1,9 et, de préférence, une proportion de blocks (G) supérieure à 5Ø %, le (ou les) alginate(s) étant, de préférence un (ou des) alginate(s) de sodium ayant une viscosité en solution aqueuse à Ø,5 % en poids à 25°C inférieure à 2Ø mPa.s mesurée au viscosimètre TV Contraves avec un corps de mesure n° 1 en présence d'un chélatant du calcium ;
b) la concentration pondérale en alginate(s) de la solution aqueuse introduite par la (ou les) buse(s) est comprise entre Ø,2 et 2 %, de préférence entre Ø,3 et 1 % ;
c) la concentration molaire en cation métallique polyvalent de la solution aqueuse réticulante de sel(s) de métal polyvalent est de 3,4 x 1Ø$^{-3}$M à 6,8 x 1Ø$^{-2}$M, de préférence 6,8 x 1Ø$^{-3}$M à 3,4 x 1Ø$^{-2}$M ;
d) la tension superficielle de la solution aqueuse de sel(s) de métal polyvalent est abaissée à une valeur inférieure à 7Ø dynes/cm, de préférence voisine de 4Ø dynes/cm, par addition d'un agent tensio-actif ; et
e) le temps de contact entre les gouttes de solution d'alginate(s) et la solution aqueuse de sel(s) de métal polyvalent est un temps précis compris entre 1Ø secondes et 2Ø minutes, de préférence entre 3Ø secondes et 1Ø minutes.

2. Procédé selon la revendication 1, caractérisé par le fait que la solution aqueuse d'alginate(s) contient au moins un additif ou actif cosmétique hydrosoluble ou dispersible.

3. Procédé selon les revendications 1 ou 2, caractérisé par le fait que la solution aqueuse d'alginate(s) contient une faible quantité d'ions réticulants.

4. Procédé selon l'une des revendications 1 à 3, caractérisé par le fait que la solution aqueuse de sel(s) de métal polyvalent utilisée est une solution de chlorure, de gluconate ou de lactate de calcium ; d'acétate, de sulfate, de chlorure ou de gluconate de cuivre ou de zinc.

5. Procédé selon l'une des revendications 1 à 4, caractérisé par le fait que la densité de la solution d'alginate(s) est légèrement supérieure à celle de la solution des cations polyvalents.

6. Procédé selon l'une des revendications 1 à 5, caractérisé par le fait que la solution d'alginate(s) est délivrée au-dessus de la solution de sel(s) de métal polyvalent.

7. Procédé selon l'une des revendications 1 à 5, caractérisé par le fait que la solution d'alginate(s) est injectée sous la surface de la solution réticulante.

8. Procédé selon l'une des revendications 1 à 7, caractérisé par le fait au'après rinçage et séchage, on enrobe les capsules d'alginate(s) par une couche d'un polymère plus ou moins adhésif en solution ou en suspension.

9. Appareil pour la mise en oeuvre du procédé selon l'une des revendications 1 à 8, caractérisé par le fait qu'il comporte un réservoir de stockage de la solution d'alginate(s), au moins une buse d'injection, une pompe prélevant la solution d'alginate(s) du réservoir de stockage et alimentant la (ou les) buse(s), un bac allongé alimenté en continu à une de ses extrémités par une solution de sel(s) de cation réticulant et comportant, à son autre extrémité, un dispositif de soutirage de ladite solution de sel(s) de métaux polyvalents qui est évacuée ou régénérée et recyclée, la solution d'alginate(s) tombant goutte à goutte de la (ou des) buse(s) dans la solution de sel(s) de cation réticulant, à l'extrémité du bac correspondant à l'alimentation en ce(s) sel(s), et un dispositif de transport disposé longitudinalement dans le bac et permettant de transporter les capsules d'alginate(s) en cours de formation d'une extrémité à l'autre du bac.

10. Appareil selon la revendication 9, caractérisé par le fait que le dispositif de transport est une bande sans fin ou une vis sans fin.

11. Appareil selon la revendication 1∅, caractérisé par le fait que la bande sans fin est formée d'une trame dont la maille est inférieure au diamètre des gouttes tombant de la buse.

12. Appareil selon la revendication 11, caractérisé par le fait que la bande sans fin comporte des saillies transversales.

13. Composition cosmétique contenant les capsules d'alginate(s) préparées selon le procédé de l'une des revendications 1 à 8, dans une phase externe cosmétiquement acceptable.

## Claims

1. Process for the manufacture of capsules of alginate(s) by introducing dropwise, through at least one nozzle, an aqueous solution of at least one alginate into an aqueous solution of at least one polyvalent metal salt, the said metal being selected from iron, silver, strontium, aluminium, manganese, selenium, and in particular calcium, copper and zinc, and then extracting the capsules of alginate(s), formed by gelling, so as to separate them from the aqueous solution of polyvalent metal salt(s) and finally, optionally, washing and drying the said capsules, characterised in that:

a) at least one alginate is used which has mannuronic (M) and guluronic (G) units in an M/G molar ratio between 0.4 and 1.9 and, preferably, a proportion of (G) blocks greater than 50 %, the alginate(s) being preferably sodium alginate(s) having a viscosity in aqueous solution, at 0.5 % by weight and at 25°C, less than 20 mpa.s measured with a TV Contraves viscosimeter provided with a No. 1 measurement body in the presence of a calcium-chelating agent;

b) the weight concentration of alginate(s) in the aqueous solution introduced through the nozzle(s) is between 0.2 and 2 %, preferably between 0.3 and 1 %;

c) the molar concentration of polyvalent metal cation in the cross-linking aqueous solution of polyvalent metal salt(s) is from $3.4 \times 10^{-3}$ M to $6.8 \times 10^{-2}$ M, preferably $6.8 \times 10^{-3}$ M to $3.4 \times 10^{-2}$ M;

d) the surface tension of the aqueous solution of polyvalent metal salt(s) is reduced to a value less than 70 dynes/cm, preferably about 40 dynes/cm, by the addition of a surface-active agent; and

e) the contact time between the drops of solution of alginate(s) and the aqueous solution of polyvalent metal salt(s) is a defined period of time between 10 seconds and 20 minutes, preferably between 30 seconds and 10 minutes.

2. Process according to Claim 1, characterised in that the aqueous solution of alginate(s) contains at least one water-soluble or one dispersible cosmetic additive or active agent.

3. Process according to Claims 1 or 2, characterised in that the aqueous solution of alginate(s) contains a small amount of cross-linking ions.

4. Process according to one of Claims 1 to 3, characterised in that the aqueous solution of polyvalent metal salt(s) used is a solution of calcium chloride, gluconate or lactate; or of copper or zinc acetate, sulphate, chloride or gluconate.

5. Process according to one of Claims 1 to 4, characterised in that the density of the solution of alginate(s) is slightly greater than that of the solution of polyvalent cations.

6. Process according to one of Claims 1 to 5, characterised in that the solution of alginate(s) is delivered from above the solution of polyvalent metal salt(s).

7. Process according to one of Claims 1 to 5, characterised in that the solution of alginate(s) is injected under the surface of the cross-linking solution.

8. Process according to one of Claims 1 to 7, characterised in that after rinsing and drying, the capsules of alginate(s) are coated with a layer of a more or less adhesive polymer in solution or in suspension.

9. Apparatus for performing the process according to one of Claims 1 to 8, characterised in that it comprises a storage reservoir for the solution of alginate(s), at least one injection nozzle, a pump drawing the solution of alginate(s) from the storage reservoir and supplying the nozzle(s), an elongated tank supplied continuously, at one of its ends, with a solution of crows-linking cation salt(s) and containing, at its other end, a device for drawing off the said solution of polyvalent metal salt(s) which is evacuated or regenerated and recycled, the solution of alginate(s) dripping from the nozzle(s) into the solution of cross-linking cation salt(s), at the end of the tank corresponding to the supply in this salt or these salts, and a transport device placed longitudinally in the tank and enabling the capsules of alginate(s) being formed to be transported from one end of the tank to the other.

10. Apparatus according to Claim 9, characterised in that the transport device is an endless belt or an endless screw.

11. Apparatus according to Claim 10, characterised in that the endless belt is formed by a frame whose mesh is smaller than the diameter of the drops dripping from the nozzle.

12. Apparatus according to Claim 11, characterised in that the endless belt comprises transverse projections.

13. Cosmetic composition containing the capsules of alginate(s) prepared according to the process of one of Claims 1 to 8, in a cosmetically acceptable external phase.

**Patentansprüche**

1. Verfahren zur Herstellung von Alginatkapseln durch tropfenweise Einleitung einer wäßrigen Lösung wenigstens eines Alginates in eine wäßrige Lösung wenigstens eines Salzes eines polyvalenten Metalls, ausgewählt unter Eisen, Silber, Strontium, Aluminium, Mangan, Selen und insbesondere Calcium, Kupfer und Zink mit Hilfe von wenigstens einer Spritzdüse, anschließende Extraktion der durch Gelbildung gebildeten Alginatkapseln, um diese von der wäßrigen Lösung des Salzes (der Salze) des polyvalenten Metalls abzutrennen, und schließlich gegebenenfalls durch Waschen und Trocknen der Kapseln, dadurch gekennzeichnet, daß:

a) man wenigstens ein Alginat verwendet, welches Mannuron (M)- und Glucuron (G)-Einheiten in einem molaren Verhältnis M/G von 0,4 - 1,9 und vorzugsweise einen Anteil von Blöcken (G) über 50% aufweist, wobei das (oder die) Alginat(e) vorzugsweise Natriumalginat(e) ist (sind) mit einer Viskosität in wäßriger Lösung von 0,5 Gew.-% bei 25°C von kleiner als 20 mPa·s, bestimmt mit Hilfe des TV Contraves-Viskosimeters mit einem Meßkörper Nr.1 in Gegenwart eines Calciumchelatbildners;

b) die gewichtsbezogene Alginat-Konzentration der durch die Spritzdüse(n) eingeleiteten wäßrigen Lösung 0,2 - 2%, vorzugsweise 0,3 - 1%, beträgt;

c) die molare Konzentration an polyvalentem Metallkation in der vernetzenden wäßrigen Lösung des Salzes (der Salze) des polyvalenten Metalls $3,4 \times 10^{-3}$ M bis $6,8 \times 10^{-2}$ M, vorzugsweise $6,8 \times 10^{-3}$ M bis $3,4 \times 10^{-2}$ M beträgt;

d) die Oberflächenspannung der wäßrigen Lösung des Salzes (der Salze) des polyvalenten Metalls auf einen Wert unterhalb von 70 dyn/cm, vorzugsweise auf etwa 40 dyn/cm, durch Zugabe eines oberflächenaktiven Mittels herabgesetzt ist; und

e) die Kontaktzeit zwischen den Tropfen der Alginatlösung und der wäßrigen Lösung des Salzes (der Salze) des polyvalenten Metalls eine bestimmte Zeit zwischen 10 Sekunden und 20 Minuten, vorzugsweise zwischen 30 Sekunden und 10 Minuten, ist.

2. Verfahren nach Anspruch 1, dadurch gekennzeichnet, daß die wäßrige Alginatlösung wenigstens einen kosmetischen, wasserlöslichen oder dispergierbaren Zusatz oder Wirkstoff enthält.

3. Verfahren nach Anspruch 1 oder 2, dadurch gekennzeichnet, daß die wäßrige Alginatlösung eine geringe Menge vernetzender Ionen enthält.

4. Verfahren nach einem der Ansprüche 1 bis 3, dadurch gekennzeichnet, daß die wäßrige Lösung des Salzes (der Salze) des verwendeten polyvalenten Metalls eine Lösung von Calciumchlorid, -gluconat oder -lactat; Kupfer- oder Zinkacetat, -sulfat, -chlorid- oder -gluconat ist.

5. Verfahren nach einem der Ansprüche 1 bis 4, dadurch gekennzeichnet, daß die Dichte der Alginatlösung geringfügig über derjenigen der Lösung der polyvalenten Kationen liegt.

6. Verfahren nach einem der Ansprüche 1 bis 5, dadurch gekennzeichnet, daß die Alginatlösung oberhalb der Lösung des Salzes (der Salze) des polyvalenten Metalls abgegeben wird.

7. Verfahren nach einem der Ansprüche 1 bis 5, dadurch gekennzeichnet, daß die Alginatlösung unterhalb der Oberfläche der vernetzenden Lösung injiziert wird.

8. Verfahren nach einem der Ansprüche 1 bis 7, dadurch gekennzeichnet, daß man die Alginatkapseln nach Spülen und Trocknen mit einem Überzug aus einem in Lösung oder in Suspension mehr oder weniger klebrigem Polymer einkapselt.

9. Vorrichtung zur Durchführung des Verfahrens nach einem der Ansprüche 1 bis 8, dadurch gekennzeichnet, daß es umfaßt: ein Speicherresevoir für die Alginatlösung; mindestens eine Spritzdüse; eine Pumpe, welche die Alginatlösung aus dem Speicherreservoir abzieht und die Spritzdüse(n) speist; einen langgestreckten Trog in dem an einem Ende kontinuierlich eine Lösung des Salzes (der Salze) der vernetzenden Kationen eingespeist wird und der an seinem anderen Ende eine Vorrichtung zum Abziehen der Lösung des Salzes (der Salze) der polyvalenten Metalle umfaßt, welche evakuiert oder regeneriert und rezykliert wird, wobei die Alginatlösung tropfenweise aus der (den) Spritzdüse(n) in die Lösung des Salzes (der Salze) des vernetzenden Kations an dem Ende des Troges fällt, an dem das Salz (die Salze) eingespeist werden; und eine Transportvorrichtung, die in dem Trog längs angeordnet ist und den Transport der Alginatkapseln im Verlauf ihrer Bildung von einem zum anderen Ende des Troges ermöglicht.

10. Vorrichtung nach Anspruch 9, dadurch gekennzeichnet, daß die Transportvorrichtung ein Endlos-Band oder eine Schnecke ist.

11. Vorrichtung nach Anspruch 10, dadurch gekennzeichnet, daß das Endlos-Band gitterförmig ausgebildet ist, dessen Maschen kleiner als der Durchmesser der aus der Spritzdüse fallenden Tropfen ist.

12. Vorrichtung nach Anspruch 11, dadurch gekennzeichnet, daß das Endlos-Band transversale Nocken umfaßt.

13. Kosmetisches Mittel, enthaltend die Alginatkapseln, hergestellt nach einem Verfahren gemäß einem der Ansprüche 1 bis 8 in einer kosmetisch verträglichen externen Phase.

FIG. 1

FIG. 2

FIG. 3

FIG. 4

FIG. 5